# EUROPEAN PATENT SPECIFICATION

(11) **EP 3 906 891 B1**
(45) Date of publication and mention of the grant of the patent: **11.11.2026**
(21) Application number: 21170611.4
(22) Date of filing: 27.04.2021
(51) Int. Cl.: A61F 2/00

(54) **IMPLANTABLE PROTHESIS FOR MINIMALLY INVASIVE HERNIA REPAIR**
IMPLANTIERBARE PROTHESE FÜR MINIMALINVASIVE HERNIEREPARATUR
PROTHÈSE IMPLANTABLE POUR LA RÉPARATION DE HERNIES MINIMALEMENT INVASIVE

(30) Priority: 28.04.2020 US 202063016750 P
(43) Date of publication of application: 10.11.2021
(73) Proprietor: Covidien LP, Mansfield, MA 02048 (US)
(72) Inventor: Sneider, Mark S., Golden Valley, Minnesota 55416 (US)
(74) Representative: Viering, Jentschura & Partner mbB Patent- und Rechtsanwälte

(56) References cited:
- EP-A1- 0 836 838
- EP-A1- 3 398 554
- US-A1- 2013 178 875
- US-A1- 2019 117 363

## Description

### BACKGROUND

### Technical Field

The instant disclosure relates to an implantable prothesis for use in hernia repair surgery. In particular, the implantable prothesis can be used during minimally invasive surgical repair of inguinal hernias, including direct, indirect, and bilateral hernia defects.

### Related Art

Surgically implantable prosthetic mesh patches ("prosthetic mesh," "implantable mesh," "implantable prosthesis," "mesh patch," "mesh," and/or the like) are commonly used for the repair of inguinal and other abdominal wall hernias. Currently available mesh patches designed for minimally invasive (e.g., laparoscopic or robotic) inguinal hernia repair are either flat sheets of mesh or have a three-dimensional shape that conforms to the groin anatomy. Such mesh patches typically come in three different sizes (medium, large, and extra-large), and they are generally similar in shape. Examples of currently available hernia repair mesh patches products include 3D Max^{™} manufactured by Bard and Dextile^{™} manufactured by Medtronic. These products are designed to conform to the inguinal anatomy and cover the myopectineal orifice, but they are not specifically or preferentially designed for either the direct, or indirect hernia spaces.

The foregoing discussion is intended only to illustrate the present field and should not be taken as a disavowal of claim scope. European Patent Application EP3398554 describes a prosthesis for inguinal hernia repair formed from a piece of biocompatible material having a pre-formed 3-D shape. US Patent Application US 2019/117363 describes a self-gripping hernia prosthesis including a layer of repair fabric and a plurality of tissue grips, European Patent Application EP0836838 describes a prosthesis for the repair of hernias, and US Patent Application US 20130178875 describes an implantable prosthesis having a 3-D shape which is invertible.

### SUMMARY

The present disclosure describes both unilateral and bilateral implantable prostheses designed for hernia repair and particularly inguinal hernia repair. The invention is defined in claim 1. Further preferred aspects are defined in the dependent claims.

In a first embodiment is provided an implantable prosthesis for repairing a direct or indirect inguinal hernial defect comprising:
a piece of biocompatible material having a preformed three-dimensional shape and defined by an outer edge , the piece having a lateral side and an opposite medial side at opposite ends of a medial-lateral axis, and a superior side and an opposite inferior side at opposite ends of a superior-inferior axis, and the outer edge including a convex lateral outer edge, a medial outer edge, a superior outer edge configured to widen the piece in a medial direction, and an inferior outer edge including one or more concave portions, the piece including:
an upper portion including a spherical cap extending between the superior outer edge and an interior edge defining a crease or fold extending from the lateral side to the medial side, the interior edge including first and second bifurcated portions on a medial end thereof,
a lower portion extending between the interior edge and the inferior outer edge, the lower portion forming a wavy-shaped wall, and
a crease portion on the medial side, the crease portion defined between the first and second bifurcated end portions on the medial end thereof, characterized in that at least one of the first and second bifurcated portions of the interior edge are non-linear and do not extend to the medial outer edge.

The superior outer edge may be generally linear.

The piece may further comprise a longest length and a widest width, the longest length extending between the convex lateral outer edge and the medial outer edge and the widest width located on the medial side of the piece extending between the superior outer edge and the inferior outer edge.

The interior edge may extend from a portion of the convex lateral edge positioned inferior to the longest length.

The interior edge may be a non-linear interior edge extending along or crossing over at least a portion of the longest length.

The medial outer edge may be a concave medial outer edge.

The implantable prosthesis may further comprise one or more barbs positioned on a face of the piece.

The one or more barbs may be positioned on at least a portion of the medial side of the piece along the medial outer edge, positioned on at least a portion of the lower portion of the piece along the inferior outer edge, or both.

The implantable prosthesis may further comprise a second section, the second section including:
a second lateral side opposite a second medial side extending along a second medial-lateral axis of the second section and a second superior side opposite a second inferior side extending along a second superior-inferior axis of the second section,
a second upper portion including a spherical cap extending between a second portion of the superior outer edge and a second interior edge extending from the second lateral side to the second medial side, the second interior edge including a third and fourth bifurcated portion on a medial end thereof,
a second lower portion extending between the second interior edge and a second portion of the inferior outer edge, the second lower portion forming a second wavy-shaped wall,
a second crease portion on the second medial portion and defined between the third and fourth bifurcated portions of the second crease on the medial end thereof,
wherein the first and second medial sides of the first and second sections form a central band between the first and second lateral sides across a face of the piece of biocompatible material.

### BRIEF DESCRIPTION OF THE DRAWINGS

The following figures are included to illustrate certain aspects of the present invention and should not be viewed as exclusive embodiments. The subject matter disclosed is capable of considerable modifications, alterations, and combinations, as within the scope of the appended claims.
Fig. 1 is a plan view of an embodiment of an implantable prosthesis designed for repair of a right-sided direct hernia.
Fig. 2 is a cross-sectional view along the superior-inferior axis B₁ of the implantable prosthesis shown in Fig. 1.
Fig. 3 is an interior view of the implantable prosthesis shown in Fig. 1 within an associated anatomical context.
Fig. 4 is a plan view of an embodiment of an implantable prosthesis designed for repair of a left-sided direct hernia.
Fig. 5 is a cross-sectional view along the superior-inferior axis B₂ of the implantable prosthesis shown in Fig. 4.
Fig. 6 is an interior view of the implantable prosthesis shown in Fig. 4 within an associated anatomical context.
Fig. 7 is a plan view of an embodiment of an implantable prosthesis designed for repair of a right-sided indirect hernia.
Fig. 8 is a cross-sectional view along the superior-inferior axis B₃ of the implantable prosthesis shown in Fig. 7.
Fig. 9 is an interior view of the implantable prosthesis shown in Fig. 7 within an associated anatomical context.
Fig. 10 is a plan view of an embodiment of an implantable prosthesis designed for repair of a left-sided indirect hernia.
Fig. 11 is a cross-sectional view along the superior-inferior axis B₄ of the implantable prosthesis shown in Fig. 10.
Fig. 12 is an interior view of the implantable prosthesis shown in Fig. 10 within an associated anatomical context.
Fig. 13 is a plan view of an embodiment of an implantable prosthesis designed for repair of a bilateral direct hernias.
Fig. 14A is a cross-sectional view of the first section of the implantable prosthesis along the superior-inferior axis B₅ shown in Fig. 13.
Fig. 14B is a cross-sectional view of the second section of the implantable prosthesis along the superior-inferior axis B₆ shown in Fig. 13.
Fig. 15 is an interior view of the implantable prosthesis shown in Fig. 13 within an associated anatomical context.
Fig. 16 is a plan view of an embodiment of an implantable prosthesis designed for repair of a bilateral indirect hernias.
Figs. 17A and 17 B are a plan view of an embodiment of an implantable prosthesis designed for repair of a bilateral hernia including a direct and indirect hernia.
Fig. 18 is a plan view of an embodiment of a self-fastening implantable prosthesis designed for repair of a bilateral hernia.
Fig. 19 is a plan view of an embodiment of a first and second unilateral implantable prosthesis being combined to form a bilateral implantable prosthesis designed for repair of a bilateral hernia.

### DETAILED DESCRIPTION OF EMBODIMENTS

Several embodiments of implantable prostheses are disclosed herein. Details of the various embodiments of the present disclosure are described below with specific reference to the figures.

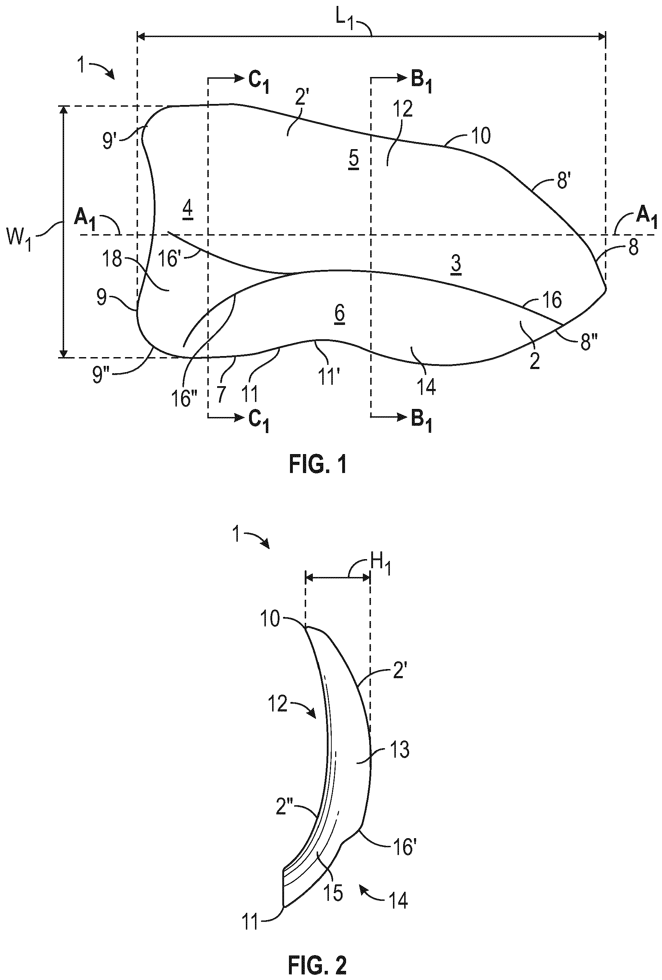

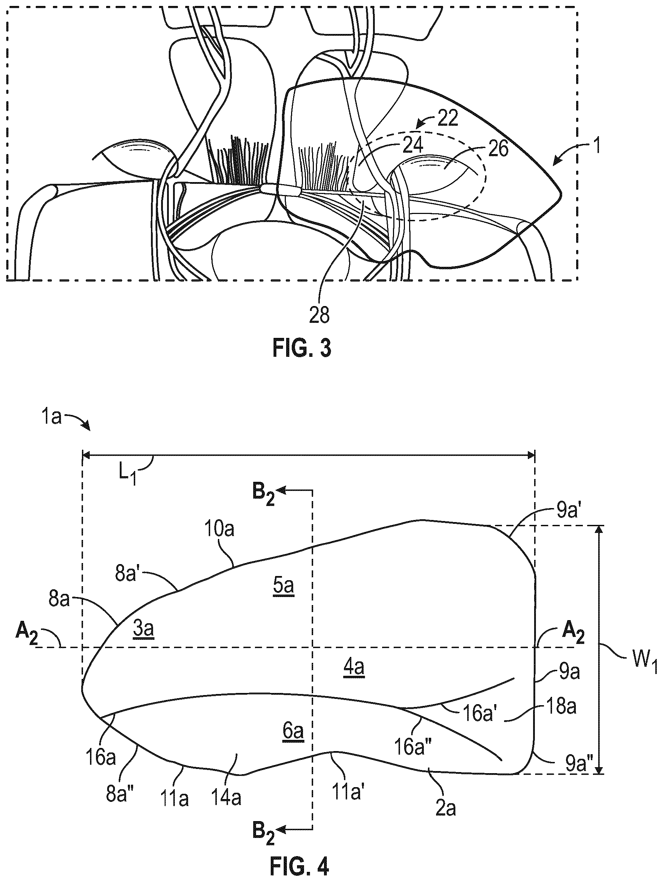

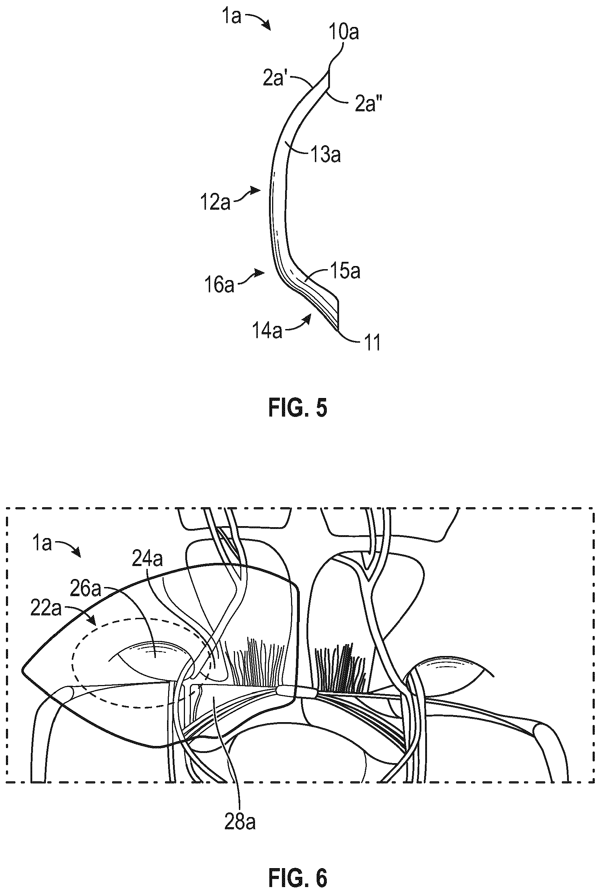

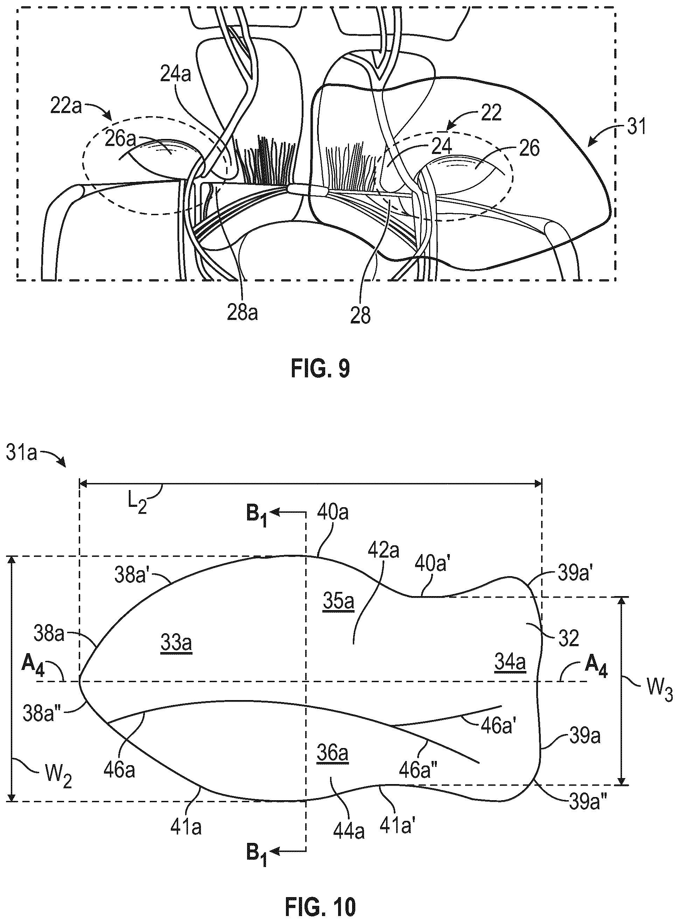

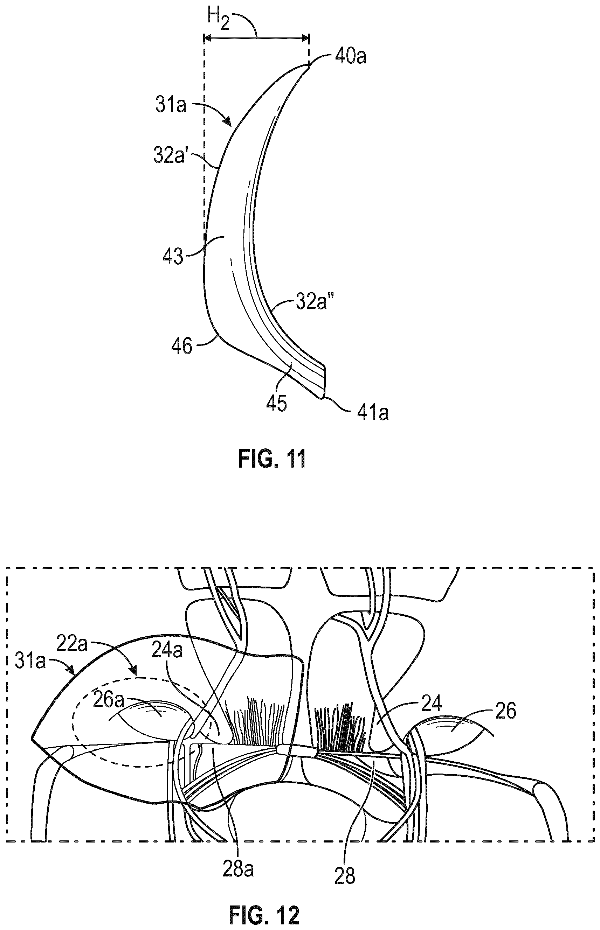

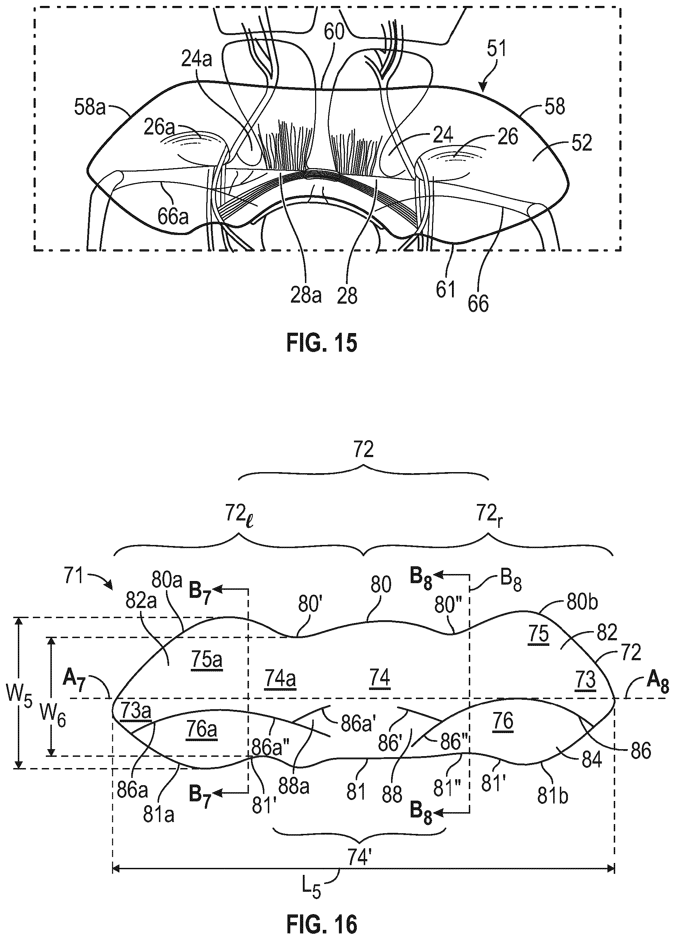

Figs. 1-19 illustrate various embodiments of an implantable prosthesis that is specifically designed for minimally invasive, robotic, or laparoscopic, repair of one or more defects in muscle or tissue wall, such as a hernia. In particular, the various embodiments of the implantable prosthesis described herein are tailored for inguinal hernia repair, including direct, indirect, and bilateral hernia defects. Direct inguinal hernias can be defined as hernias that originate through the floor of the inguinal canal, through the transversalis fascia, medial to the epigastric vessels (shown and described below with respect to Figs. 3 and 6). Indirect inguinal hernias can be defined as hernias that occur through a defect lateral to the epigastric vessels, through the internal inguinal ring (shown and described below with respect to Fig. 9 and 12). Bilateral inguinal hernias can be defined as hernias that are present in both inguinal spaces, right and left (shown and described below with respect to Fig. 15) and can be a combination of direct and indirect hernias. For example, one side can have a direct hernia and the contralateral side can be indirect, or both can be the same type of hernia.

The implantable prostheses described herein can be made of any biocompatible material. The term biocompatible is understood as meaning that the materials can be safely implanted in the human or animal body. Biocompatible materials may include bioresorbable materials, non-bioresorbable materials, and combinations thereof.

Some non-limiting examples of bioresorbable material suitable for the piece of the prostheses described herein can be chosen from among the following materials: polylactic acid (PLA), polycaprolactones (PCL), polydioxanones (PDO), trimethylene carbonates (TMC), polyvinyl alcohol (PVA), polyhydroxyalkanoates (PHA), oxidized cellulose, polyglycolic acid (PGA), polyethylene glycol (PE), copolymers of these materials, and mixtures thereof.

Some non-limiting examples of non-bioresorbable material suitable for the piece of the prostheses described herein can be chosen from among the following non-bioresorbable materials: polypropylenes, polyesters such as polyethylene terephthalates, polyamides, silicones, polyether ether ketone (PEEK), polyarylether ether ketone (PAEK), and mixtures thereof.

The biocompatible piece may be a porous material. In some embodiments, the biocompatible piece of material may be a surgical mesh, in particular a porous surgical mesh. The piece and/or surgical mesh may include any arrangement or assembly of biocompatible yarns, fibers, filaments and/or multifilaments, for example obtained by knitting, weaving, braiding, or even non-woven. The biocompatible pieces and/or surgical mesh, in particular porous pieces and/or surgical mesh, are suitable for the repair of a hernia defect, and particularly repair of an inguinal hernia defect.

In some embodiments, an implantable surgical mesh made of polypropylene may have a single layer monofilament weave with a large pore size (greater than about 1 mm) and a medium (standard) density between about 70-140 g/m². Further, it may be non-absorbable. The mesh can have reinforced edges (e.g., by heat sealing) to give it firmness and to avoid fraying and unraveling of the material as it is handled during the procedure.

The implantable prostheses described herein can have a variety of shapes and sizes, as further described below, and it can have a three-dimensional (3D) contour allowing them to conform to the inguinal canal anatomy (although flat contours may also be used). The prostheses can be designed to fit in the right or left inguinal space, with a specific shape that provides preferential coverage to the floor of the groin to ideally repair direct inguinal hernia defects or larger coverage laterally to provide better repair indirect inguinal hernia defects. The implantable prostheses can also cover the entire myopectineal orifice, which is the entire inguinal space from which both direct and indirect hernias occur. Further, the prostheses can be amenable to being rolled up into a cylindrical shape and inserted through a trocar or other surgical device into the abdomen or preperitoneal space. A surgeon can use a grasper tool or instrument to hold an edge of the prostheses and insert it into the appropriate body cavity by rolling it into a cylinder or scroll shape, or simply pushing it in, depending on the size of the trocar that is used for introducing the prostheses. After insertion, the prostheses can expand and return to its original 3D-conforming shape.

Turning now to Figs. 1-3, an implantable prosthesis 1 designed for right-sided direct hernia is shown. The implantable prosthesis 1 includes a piece 2 of biocompatible material, such as a textile or mesh, defined by an outer edge 7. The prosthesis 1 (and/or biocompatible piece 2) displays a three-dimensional shape (Fig. 2).

Fig. 1 depicts a medial-lateral axis A₁ and a superior-inferior axis B₁ across a first face 2' of the biocompatible piece 2 (and/or prosthesis 1). The biocompatible piece 2 has a lateral side 3, a medial side 4, a superior side 5, and an inferior side 6. The piece 2 also includes a second face 2" opposite the first face 2' (Fig. 2).

The outer edge 7 of the biocompatible piece 2 includes a convex lateral outer edge 8, a medial outer edge 9, a superior outer edge 10, and a concave inferior outer edge 11. The convex lateral outer edge 8 is opposite the medial outer edge 9 along the medial-lateral axis A₁. The superior outer edge 10 is opposite the concave inferior edge 11 along the superior-inferior axis B₁. The concave inferior outer edge 11 includes at least one concavity 11'. The inferior outer edge 11 connects an inferior portion 8" of the convex lateral outer edge 8 to an inferior portion 9" of the medial outer edge 9. The superior outer edge 10 connects a superior portion 8' of the convex lateral outer edge 8 to a superior portion 9' of the medial outer edge 9.

As depicted in Fig. 1, in some embodiments, the outer edge 7 of the biocompatible piece 2 includes a convex lateral outer edge 8, a concave medial outer edge 9, a concave inferior outer edge 11, and superior outer edge 10 configured to widen the piece 2 in a medial direction. In such embodiments, the widening superior outer edge 10 may be of any configuration (e.g., one or more generally linear edges, convex edges, or combinations thereof) suitable for rendering the medial side 4 of the piece 2 (and/or prosthesis 1) wider than the lateral side 3.

As further depicted in Fig.1, in some embodiments, the biocompatible piece 2 is configured to repair a hernia, such as a direct inguinal hernia, and includes a longest length L₁ and a widest width W₁. The longest length L₁ extends between the convex lateral outer edge 8 and the medial outer edge 9. The widest width W₁ is located on the medial side 4 of the piece 2 of the prosthesis 1 designed for direct inguinal hernia repair. The widest width W₁ extends between the superior outer edge 10 and the wavy inferior outer edge 11.

The longest length L₁ of the piece 2 (and/or prosthesis 1) can be between about 14 cm and about 17 cm, and the widest width W₁ of the piece 2 (and/or prosthesis 1) can be between about 10 cm and about 12 cm. For example, a medium size direct hernia prosthesis 1 can be about 14 cm x 10 cm (L₁ x W₁). A large size direct hernia prosthesis 1 can be about 16 cm x 11 cm (L₁ x W₁). Finally, an extra-large size direct hernia prosthesis 1 can be 17 cm by 12 cm (L₁ x W₁).

The biocompatible piece 2 also includes an upper portion 12, a lower portion 14, an interior edge 16 extending from the lateral side 3 to the medial side 4, and a crease portion 18. The upper portion 12 extends from the lateral side 3 to the medial side 4 across at least the superior side 5 of the piece 2. The upper portion 12 also defines a curved body 13 having a height H₁ transverse to the length and width (Figs. 2 and 5). The curved body 13 (and/or upper body portion 12) begins at the superior outer edge 10 and extends with a raised curved trajectory towards the interior edge 16 and to a height H₁ of the interior edge 16. The curved body 13 (and/or upper portion 12) may also radiate medially along the superior outer edge 10 to at least the superior portion 9' of the medial outer edge 9 and/or radiate laterally along the superior outer edge 10 to at least the superior portion 8' of the convex lateral outer edge 8. In some embodiments, the upper portion 12 may be a spherical cap.

The lower portion 14 defines a wavy-shaped wall 15 extending between the interior edge 16 and the concave inferior outer edge 11. The lower portion 14 extends between the lateral side 3, and particularly the inferior portion 8" of the convex lateral outer edge 8, to the medial side 4, and particularly the inferior portion 9" of the medial outer edge 9.

The interior edge 16 defines a crease or fold extending from the lateral side 3 to the medial side 4. As shown in Fig. 1, the interior edge 16 extends from or near a portion of the convex lateral outer edge 8 positioned inferior to the longest length L₁ of the piece 2. The interior edge 16 may be rounded or non-linear, as shown, extending along and/or across at least a portion of the longest length L₁.

The interior edge 16 includes a first and second bifurcated end portion 16', 16" on the medial side 4 of the piece 2. The first and second bifurcated end portions 16', 16" generally define the crease portion 18 therebetween, with or without a portion of the medial outer edge 9, or particularly the inferior portion 9" of the medial edge 9. In some embodiments, at least one, if not both, of the first and second bifurcated end portions 16', 16" of the interior edge 16 are rounded or non-linear. In some embodiments, at least one, if not both, of the first and second bifurcated end portions 16', 16" of the interior edge 16 may not extend to the medial outer edge 9.

The upper portion 12 of the piece 2 may have a surface area which is greater than a surface area of the lower portion 14, the crease portion 18, or both. The upper portion 12 may represent a majority of the surface area of the piece 2. In some embodiments, the upper portion 12 may represent from about 50% to about 80% of the surface area of the piece 2. In some embodiments, the upper portion 12 may represent from about 55% to about 75% of the surface area of the piece 2. In some embodiments, the upper portion 12 may represent from about 60% to about 70% of the surface area of the piece 2.

The lower portion 14 of the piece 2 may have a surface area which is less than a surface area of the upper portion 12, or greater than a surface area of the crease portion 18, or both. The lower portion 14 may represent from about 15% to about 45% of the surface area of the piece 2. In some embodiments, the lower portion 14 may represent from about 20% to about 40% of the surface area of the piece 2. In some embodiments, the lower portion 14 may represent from about 25% to about 35% of the surface area of the piece 2.

As further illustrated in Figs. 1 and 2, the lower portion 14 of the piece 2 can have a raised, wavy section 15 designed to accommodate the iliac vessels as they course deep (posterior) to the piece 2. The first and second bifurcated end portions 16', 16"of crease 16 allows at least a portion, if not a majority, of the crease portion 18 of the piece 2 to sit in the space of Retzius, between the bladder and Cooper's ligament (shown to better advantage in Fig. 3). In addition, the curved body 13 of the upper portion 12, shown in Fig. 2, contributes to the 3D design of the prosthesis 1 and allows for an anatomical fit in the groin.

Fig. 3 illustrates the right-side direct prosthesis 1, e.g., hernia mesh, within the anatomical context in which it is designed to be implanted. The prosthesis 1 covers the right myopectineal orifice 22 (dashed line) of the groin, which includes the entire right inguinal space. The "direct" nature of the prosthesis 1 offers preferential coverage over the floor of the right inguinal canal (Hesselbach's triangle) 24, from which direct hernias originate. For example, the widest width W₁ of the direct prosthesis 1 is located on the medial side 4 of the prosthesis 1. Also shown in Fig. 3 is the right internal inguinal ring 26, from which indirect inguinal hernias originate, as well as the right femoral space 28, where femoral hernias can occur through the femoral canal (a space bordered by the inguinal ligament superiorly, femoral vein laterally, and Cooper's ligament inferiorly). Implantable prostheses for both direct and indirect hernias (discussed below) can also be used for the repair of femoral hernias.

The right-side direct prosthesis 1, e.g., hernia mesh, is configured to be centered over the right iliac vessels and extend medially to provide more coverage, due to its size and shape, than what is currently offered by existing implantable products. For example, the prosthesis 1 may extend medially to the symphysis pubis. The prosthesis 1 provides greater overlap over the floor of the inguinal canal, allowing for a more adequate repair of a direct inguinal hernia. The prosthesis 1 also extends laterally and is further configured to provide the required coverage of the indirect hernia space (internal inguinal ring 26) for a complete inguinal hernia repair.

Turning now to Figs. 4-6, an embodiment of an implantable prosthesis 1a designed for a left-sided direct hernia is shown. Prosthesis 1a is largely similar to prosthesis 1, as described in detail hereinabove.

The implantable prosthesis 1a includes a piece 2a of biocompatible material, such as a textile or mesh, defined by an outer edge 7a. The prosthesis 1a (and/or biocompatible piece 2a) displays a three-dimensional shape (Fig. 5).

Fig. 4 depicts a medial-lateral axis A₂ and a superior-inferior axis B₂ across a first face 2a' of the biocompatible piece 2a (and/or prosthesis 1a). The biocompatible piece 2a has a lateral side 3a, a medial side 4a, a superior side 5a, and an inferior side 6a. The piece 2a also includes a second face 2a" opposite the first face 2a' (Fig. 5).

The outer edge 7a of the biocompatible piece 2a includes a convex lateral outer edge 8a, a medial outer edge 9a, a superior outer edge 10a, and a concave inferior outer edge 11a. The convex lateral outer edge 8a is opposite the medial outer edge 9a along the medial-lateral axis A₂. The superior outer edge 10a is opposite the wavy inferior edge 11a along the superior-inferior axis B₂. The concave inferior outer edge 11a includes at least one concavity 11a'. The inferior outer edge 11a connects an inferior portion 8a" of the convex lateral outer edge 8a to an inferior portion 9a" of the medial outer edge 9a. The superior outer edge 10a connects a superior portion 8a' of the convex lateral outer edge 8a to a superior portion 9a' of the medial outer edge 9a.

As depicted in Fig. 4, in some embodiments, the outer edge 7a of the biocompatible piece 2a includes a convex lateral outer edge 8a, a concave medial outer edge 9a, a concave inferior outer edge 11a, and superior outer edge 10a configured to widen the piece 2a in a medial direction. In such embodiments, the widening superior outer edge 10a may be of any configuration (e.g., one or more generally linear edges, convex edges, or combinations thereof) suitable for rendering the medial side 4a of the piece 2a (and/or prosthesis 1a) wider than the lateral side 3a.

As further depicted in Fig. 4, in some embodiments, the biocompatible piece 2a is configured to repair a hernia, such as a direct inguinal hernia, and includes a longest length L₁ and a widest width W₁. The longest length L₁ extends between the convex lateral outer edge 8a and the medial outer edge 9a. The widest width W₁ is located on the medial side 4a of the piece 2a of the prosthesis 1a designed for direct inguinal hernia repair. The widest width W₁ extends between the superior outer edge 10a and the inferior outer edge 11a.

The longest length L₁ of the piece 2a (and/or prosthesis 1a) can be between about 14 cm and about 17 cm, and the widest width W₁ of the piece 2a (and/or prosthesis 1a) can be between about 10 cm and about 12 cm. For example, a medium size direct hernia prosthesis 1a can be about 14 cm x 10 cm (L₁ x W₁). A large size direct hernia prosthesis 1a can be about 16 cm x 11 cm (L₁ x W₁). Finally, an extra-large size direct hernia prosthesis 1a can be 17 cm by 12 cm (L₁ x W₁).

The biocompatible piece 2a also includes an upper portion 12a, a lower portion 14a, an interior edge 16a extending from the lateral side 3a to the medial side 4a, and a crease portion 18a. The upper portion 12a extends from the lateral side 3a to the medial side 4a across at least the superior side 5a of the piece 2a. The upper portion 12a also defines a curved body 13a (Fig. 5) having a height H₁ (transverse to the length and width). The curved body 13a (and/or upper body portion 12a) begins at the superior outer edge 10a and extends with a raised curved trajectory towards the interior edge 16a and to a height H₁ of the interior edge 16a. The curved body 13a (and/or upper portion 12a) may also radiate medially along the superior outer edge 10a to at least the superior portion 9a' of the medial outer edge 9a and/or radiate laterally along the superior outer edge 10a to at least the superior portion 8a' of the convex lateral outer edge 8a. In some embodiments, the upper portion 12a may be a spherical cap.

The lower portion 14a defines a wavy-shaped wall 15a extending between the interior edge 16a and the concave inferior outer edge 11a. The lower portion 14a extends between the lateral side 3a, and particularly the inferior portion 8a" of the convex lateral outer edge 8a, to the medial side 4a, and particularly the inferior portion 9a" of the medial outer edge 9a.

The interior edge 16a defines a crease or fold extending from the lateral side 3a to the medial side 4a. As shown in Fig. 4, the interior edge 16a extends from or near a portion of the convex lateral outer edge 8a positioned inferior to the longest length L₁ of the piece 2a. The interior edge 16a may be rounded or non-linear, as shown, extending along and/or across at least a portion of the longest length L₁.

The interior edge 16a includes a first and second bifurcated end portion 16a', 16a" on the medial side 4a of the piece 2a. The first and second bifurcated end portions 16a', 16a" generally define the crease portion 18a therebetween, with or without a portion of the medial outer edge 9a, or particularly without the inferior portion 9a" of the medial edge 9a. In some embodiments, at least one, if not both, of the first and second bifurcated end portions 16a', 16a" of the interior edge 16a are rounded or non-linear. In some embodiments, at least one, if not both, of the first and second bifurcated end portions 16a', 16a" of the interior edge 16a may not extend to the medial outer edge 9a.

The upper portion 12a of the piece 2a may have a surface area which is greater than a surface area of the lower portion 14a, the crease portion 18a, or both. The upper portion 12a may represent a majority of the surface area of the piece 2a. In some embodiments, the upper portion 12a may represent from about 50% to about 80% of the surface area of the piece 2a. In some embodiments, the upper portion 12a may represent from about 55% to about 75% of the surface area of the piece 2a. In some embodiments, the upper portion 12a may represent from about 60% to about 70% of the surface area of the piece 2a.

The lower portion 14a of the piece 2a may have a surface area which is less than a surface area of the upper portion 12a, or greater than a surface area of the crease portion 18a, or both. The lower portion 14a may represent from about 15% to about 45% of the surface area of the piece 2a. In some embodiments, the lower portion 14a may represent from about 20% to about 40% of the surface area of the piece 2a. In some embodiments, the lower portion 14a may represent from about 25% to about 35% of the surface area of the piece 2a.

As further illustrated in Figs. 4 and 5, the lower portion 14a of the piece 2a can have a raised, wavy section 15a designed to accommodate the iliac vessels as they course deep (posterior) to the prosthesis 1a. The first and second bifurcated end portions 16a', 16a" of crease 16a allows at least a portion, if not a majority, of the crease portion 18a of the piece 2a to sit in the space of Retzius, between the bladder and Cooper's ligament (shown to better advantage in Fig. 6). In addition, the curved body 13a of the upper portion 12a, shown in Fig. 5, contributes to the 3D design of the piece 2a and allows for an anatomical fit in the groin.

Fig. 6 illustrates the left-side direct prosthesis 1a, e.g., hernia mesh, within the anatomical context in which it is designed to be implanted. The prosthesis 1a covers the left myopectineal orifice 22a (dashed line) of the groin, which includes the entire left inguinal space. The "direct" nature of the prosthesis 1a offers preferential coverage over the floor of the left inguinal canal (Hesselbach's triangle) 24a, from which direct hernias originate. For example, the widest width W₁ of the direct prosthesis 1a is located on the medial side 4a of the prosthesis 1a. Also shown in Fig. 6 is the left internal inguinal ring 26a, from which indirect inguinal hernias originate, as well as the left femoral space 28a, where femoral hernias can occur through the femoral canal (a space bordered by the inguinal ligament superiorly, femoral vein laterally, and Cooper's ligament inferiorly). Implantable prostheses for both direct and indirect hernias (discussed below) can be used for the repair of femoral hernias.

The left-side direct prosthesis 1a, e.g., hernia mesh, is configured to be centered over the left iliac vessels and extend medially to provide more coverage, due to its size and shape, than what is currently offered by existing implantable products. For example, the prosthesis 1a may extend medially to the symphysis pubis. The prosthesis 1a, e.g., hernia mesh, provides greater overlap over the floor of the inguinal canal, allowing for a more adequate repair of a direct inguinal hernia. The prosthesis 1a also extends laterally and is further configured to provide the required coverage of the indirect hernia space (internal inguinal ring 26a) for a complete inguinal hernia repair.

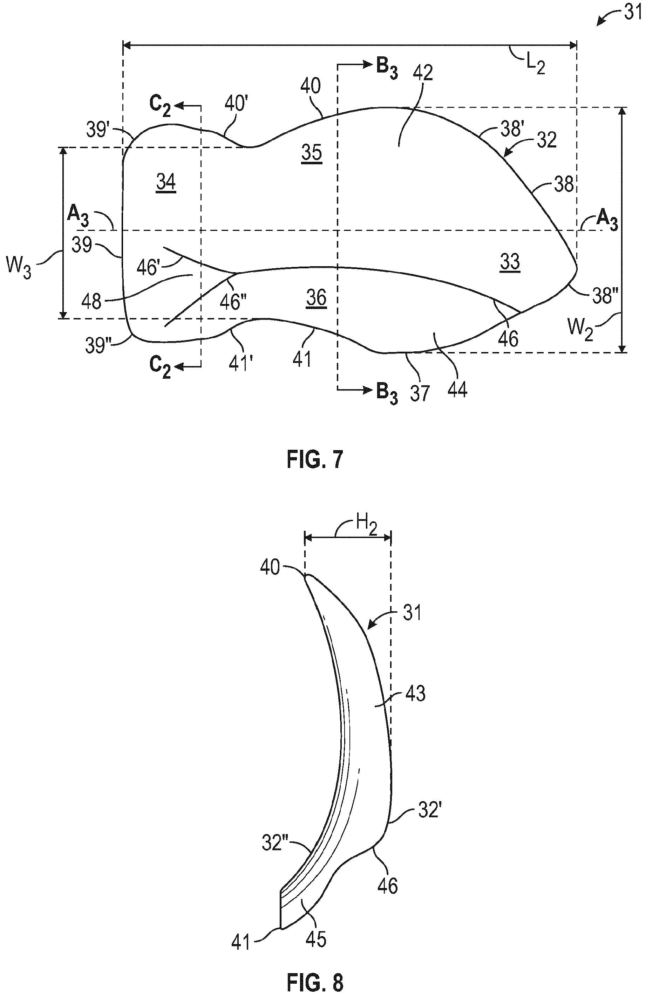

Turning now to Figs. 7-9, in some embodiments, an implantable prosthesis 31 designed for a right-sided hernia is shown. The implantable prosthesis 31 includes a piece 32 of biocompatible material, such as a textile or mesh, defined by an outer edge 37. The prosthesis 31 (and/or biocompatible piece 32) displays a three-dimensional shape (Fig. 8).

Fig. 7 depicts a medial-lateral axis A₃ and a superior-inferior axis B₃ across a first face 32' of the biocompatible piece 32 (and/or prosthesis 31). The biocompatible piece 32 has a lateral side 33, a medial side 34, a superior side 35, and an inferior side 36. The piece 32 also includes a second face 32" opposite the first face 32' (Fig. 8).

The outer edge 37 of the prosthesis 31 includes a convex lateral outer edge 38, a medial outer edge 39 opposite the lateral edge 38, a concave superior outer edge 40 connecting a superior portion 38' of the convex lateral outer edge 38 to a superior portion 39' of the medial outer edge 39, and a concave inferior outer edge 41 opposite the superior outer edge 40. The inferior outer edge 41 connecting an inferior portion 38" of the convex lateral outer edge 38 to an inferior portion 39" of the medial outer edge 39.

As depicted in Fig. 7, in some embodiments, the outer edge 37 of the prosthesis 31 includes a convex lateral outer edge 38, a generally linear or slightly concave medial outer edge 39, a concave superior outer edge 40, and a concave inferior outer edge 41. The concave portions 40', 41' of the superior and inferior outer edges 40, 41 are positioned on the medial side 34 of the piece 32 to create a shortest width W₃ between the superior and inferior outer edges 40, 41 on the medial side 34 of the piece 32.

As further depicted in Fig. 7, in some embodiments, the biocompatible piece 32 is configured to repair a hernia, such as an indirect inguinal hernia, and includes a longest length L₂ and a widest width W₂. The longest length L₂ extends between the convex lateral outer edge 38 and the medial outer edge 39. The widest width W₂ is located on the lateral side 33 of the piece 32 extending between the superior outer edge 40 and the wavy inferior outer edge 41.

The longest length L₂ of the prosthesis 31, e.g., surgical mesh, can be between about 14 cm and about 17 cm, and the widest width W₂ of the piece 32 can be between about 10 cm and about 12 cm. For example, a medium size left-side indirect hernia prosthesis 31 can be about 14 cm x 10 cm (L₂ x W₂). A large size left-side indirect hernia prosthesis 31 can be about 16 cm x 11 cm (L₂ x W₂). Finally, an extra-large size left-side indirect hernia prosthesis 31 can be 17 cm by 12 cm (L₂ x W₂).

The biocompatible piece 32 includes an upper portion 42, a lower portion 44, an interior edge 46 extending from the lateral side 33 to the medial side 34, and a crease portion 48. The upper portion 42 extends from the lateral side 33 to the medial side 34 across at least the superior side 35 of the piece 32. The upper portion 42 also defines a curved body 43 (Fig.8) having a height H₂ transverse to the length and width of the piece 32. The curved body 43 (and/or upper portion 42) begins at the superior outer edge 40 and extends with a raised curved trajectory towards the interior edge 46 and to a height H₂ of the interior edge 46. The curved body 43 (and/or upper portion 42) may also radiate medially along the superior outer edge 40 to at least the superior portion 39' of the medial outer edge 39 and/or radiate laterally along the superior outer edge 40 to at least the superior portion 38' of the convex lateral outer edge 38. In some embodiments, the upper portion 42 may be a spherical cap.

The lower portion 44 defines a wavy-shaped wall 45 extending between the interior edge 46 and the concave inferior outer edge 41. The lower portion 44 extends from the lateral side 33, and particularly from the inferior portion 38" of the convex lateral outer edge 38, to the medial side 34, and particularly not to the medial outer edge 39.

The interior edge 46 defines a crease or fold extending from the lateral side 33 to the medial side 34. As shown in Fig. 7, the interior edge 46 extends from or near a portion of the convex lateral outer edge 38 positioned inferior to the longest length L₂ of the piece 32. The interior edge 46 may be rounded or non-linear, as shown, extending along and/or across at least a portion of the longest length L₂.

The interior edge 46 includes a first and second bifurcated portion 46', 46" on the medial side 34 of the piece 32. The first and second bifurcated portions 46', 46" generally define the crease portion 48 therebetween, with or without a portion of the medial outer edge 39. In some embodiments, at least one, if not both, of the first and second bifurcated portions 46', 46" of the interior edge 46 are rounded or non-linear. In some embodiments, at least one, if not both, of the first and second bifurcated portions 46', 46" of the interior edge 46 may not extend to the medial outer edge 39.

The upper portion 42 of the piece may have a surface area which is greater than a surface area of the lower portion 44 and/or the crease portion 48. The upper portion 42 may represent a majority of the surface area of the piece 32. In some embodiments, the upper portion 42 may represent from about 50% to about 80% of the surface area of the piece 32. In some embodiments, the upper portion 42 may represent from about 55% to about 75% of the surface area of the piece 32. In some embodiments, the upper portion 42 may represent from about 60% to about 70% of the surface area of the piece 32.

As further illustrated in Figs. 7 and 8, the lower portion 44 of the prosthesis 31 (and/or piece 32) can have a raised, wavy section 45 designed to accommodate the iliac vessels as they course deep (posterior) to the prosthesis 31. The bifurcated end portions 46', 46" of the crease 46 allows at least a portion, if not a majority, of the crease portion 48 of the prosthesis 31 to sit in the space of Retzius, between the bladder and Cooper's ligament (shown to better advantage in Fig. 9). In addition, the curved body 43, shown in Fig. 8, contributes to the 3D design of the prosthesis 31 and allows for an anatomical fit in the groin.

Fig. 9 illustrates the right-side indirect prosthesis 31, e.g., hernia mesh, within the anatomical context in which it is designed to be implanted. The prosthesis 31 covers the right myopectineal orifice 22a (dashed line) of the groin, which includes the entire right inguinal space. The "indirect" nature of the prosthesis 31 offers preferential coverage laterally over the right internal inguinal ring 26a, from which indirect inguinal hernias originate. For example, the widest width W₂ of the indirect prosthesis 31a is located on the lateral side 33 of the prosthesis 31. Also, the medial side 34 of the prosthesis 31 has the smallest width W₃ to reduce the amount of biocompatible material implanted while forming at least one concavity along the superior edge 40 to avoid or reduce interaction and/or irritation with the epigastric vessels 27a. Also shown in Fig. 9 is the right inguinal canal (Hesselbach's triangle) 24a, from which direct hernias originate, as well as the right femoral space 28a, where femoral hernias can occur through the femoral canal (a space bordered by the inguinal ligament superiorly, femoral vein laterally, and Cooper's ligament inferiorly). Implantable prostheses for both direct and indirect hernias can be used for the repair of femoral hernias.

The right-side indirect prosthesis 31, e.g., hernia mesh, is configured to be centered over the right iliac vessels and extend laterally to provide more coverage, due to its size and shape, than what is currently offered by existing prosthetic products. The prosthesis 31 provides greater overlap of the internal inguinal ring 46a, allowing for a more adequate repair of an indirect inguinal hernia. The prosthesis 31 also extends medially and is further configured to provide the required coverage of the direct hernia space (floor of the inguinal canal) for a complete inguinal hernia repair.

Turning now to Figs. 10-12, in some embodiments, an implantable prosthesis 31a, e.g., hernia mesh, is designed for a left-sided indirect hernia repair. Prosthesis 31a is largely similar to prosthesis 31, as described in detail hereinabove.

Fig. 10 depicts a medial-lateral axis A₄ and a superior-inferior axis B₄ across a first face 32a' of the biocompatible piece 32a (and/or prosthesis 31a). The biocompatible piece 32a has a lateral side 33a, a medial side 34a, a superior side 35a, and an inferior side 36a. The piece 32a also includes a second face 32a" opposite the first face 32a' (Fig. 11).

The outer edge 37a of the prosthesis 31a includes a convex lateral outer edge 38a, a medial outer edge 39a opposite the lateral edge 38a, a superior outer edge 40a connecting a superior portion 38a' of the convex lateral outer edge 38a to a superior portion 39a' of the medial outer edge 39a, and a concave inferior outer edge 41a opposite the superior outer edge 40a. The concave inferior outer edge 41a includes at least one concavity 41a'. The inferior outer edge 41a connecting an inferior portion 38a" of the convex lateral outer edge 38a to an inferior portion 39a" of the medial outer edge 39a.

As depicted in Fig. 10, in some embodiments, the outer edge 37a of the prosthesis 31a includes a convex lateral outer edge 38a, a generally linear or slightly concave medial outer edge 39a, a concave superior outer edge 40a, and a concave inferior outer edge 41a. The concave portions 40a', 41a' of the superior and inferior outer edges 40a, 41a are positioned on the medial side 34a of the piece 32a to create a shortest width W₃ between the superior and inferior outer edges 40a, 41a on the medial side 34a of the piece 32a.

As further depicted in Fig. 10, in some embodiments, the biocompatible piece 32a is configured to repair a hernia, such as an indirect inguinal hernia, and includes a longest length L₂ and a widest width W₂. The longest length L₂ extends between the convex lateral outer edge 38a and the medial outer edge 39a. The widest width W₂ is located on the lateral side 33a of the piece 32a extending between the superior outer edge 40a and the inferior outer edge 41a.

The longest length L₂ of the prosthesis 31a, e.g., surgical mesh, can be between about 14 cm and about 17 cm, and the widest width W₂ of the prosthesis 31a can be between about 10 cm and about 12 cm. For example, a medium size left-side indirect hernia prosthesis 31a can be about 14 cm x 10 cm (L₂ x W₂). A large size left-side indirect hernia prosthesis 31a can be about 16 cm x 11 cm (L₂ x W₂). Finally, an extra-large size left-side indirect hernia prosthesis 31a can be 17 cm by 12 cm (L₂ x W₂).

The biocompatible piece 32a includes an upper portion 42a, a lower portion 44a, an interior edge 46a extending from the lateral side 33a to the medial side 34a, and a crease portion 48a. The upper portion 42a extends from the lateral side 33a to the medial side 34a across at least the superior side 35a of the piece 32a. The upper portion 42a also defines a curved body 43a (Fig. 11) having a height H₂ transverse to the length and width of the piece 32a. The curved body 43a (and/or upper portion 42a) begins at the superior outer edge 40a and extends with a raised curved trajectory towards the interior edge 46a and to a height H₂ of the interior edge 46a. The curved body 43a (and/or upper portion 42a) may also radiate medially along the superior outer edge 40a to at least the superior portion 39a' of the medial outer edge 39a and/or radiate laterally along the superior outer edge 40a to at least the superior portion 38a' of the convex lateral outer edge 38a. In some embodiments, the upper portion 42a may be a spherical cap.

The lower portion 44a defines a wavy-shaped wall 45a extending between the interior edge 46a and the inferior outer edge 41a. The lower portion 44a extends from the lateral side 33a, and particularly from the inferior portion 38a" of the convex lateral outer edge 38a, to the medial side 34a, and particularly not to the medial outer edge 39a.

The interior edge 46a defines a crease or fold extending from the lateral side 33a to the medial side 34a. As shown in Fig. 10, the interior edge 46a extends from or near a portion of the convex lateral outer edge 38a positioned inferior to the longest length L₂ of the piece 32a. The interior edge 46a may be rounded or non-linear, as shown, extending along and/or across at least a portion of the longest length L₂.

The interior edge 46a includes a first and second bifurcated portion 46a', 46a" on the medial side 34a of the piece 32a. The first and second bifurcated portions 46a', 46a" generally define the crease portion 48a therebetween, with or without a portion of the medial outer edge 39a. In some embodiments, at least one, if not both, of the first and second bifurcated portions 46a', 46a" of the interior edge 46a are rounded or non-linear. In some embodiments, at least one, if not both, of the first and second bifurcated portions 46a', 46a" of the interior edge 46a may not extend to the medial outer edge 39a.

The upper portion 42a of the piece may have a surface area which is greater than a surface area of the lower portion 44a and/or the crease portion 48a. The upper portion 42a may represent a majority of the surface area of the piece 32a. In some embodiments, the upper portion 42a may represent from about 50% to about 80% of the surface area of the piece 32a. In some embodiments, the upper portion 42a may represent from about 55% to about 75% of the surface area of the piece 32a. In some embodiments, the upper portion 42a may represent from about 60% to about 70% of the surface area of the piece 32a.

As further illustrated in Figs. 10 and 11, the lower portion 44 of the prosthesis 31a (and/or piece 32a) can have a raised, wavy section 45a designed to accommodate the iliac vessels as they course deep (posterior) to the prosthesis 31a. The bifurcated end portions 46a', 46a" of the crease 46a allows at least a portion, if not a majority, of the crease portion 48a of the prosthesis 31a to sit in the space of Retzius, between the bladder and Cooper's ligament (shown to better advantage in Fig. 12). In addition, the curved body 43a, shown in Fig. 11, contributes to the 3D design of the prosthesis 31a and allows for an anatomical fit in the groin.

Fig. 12 illustrates the left-side indirect hernia prosthesis 31a, e.g., surgical mesh, within the anatomical context in which it is designed to be implanted. The prosthesis 31a covers the left myopectineal orifice 42a (dashed line) of the groin, which includes the entire left inguinal space. The "indirect" nature of the prosthesis 31a offers preferential coverage laterally over the left internal inguinal ring 46a, from which indirect inguinal hernias originate. Also shown in Fig. 12 is the left inguinal canal (Hesselbach's triangle) 44a, from which direct hernias originate, as well as the left femoral space 48a, where femoral hernias can occur through the femoral canal (a space bordered by the inguinal ligament superiorly, femoral vein laterally, and Cooper's ligament inferiorly). Implantable prostheses for both direct and indirect hernias can be used for the repair of femoral hernias.

The left-side indirect hernia prosthesis 31a is configured to be centered over the left iliac vessels and extend laterally to provide more coverage, due to its size and shape, than what is currently offered by existing mesh products. The prosthesis 31a provides greater overlap of the internal inguinal ring 46a, allowing for a more adequate repair of an indirect inguinal hernia. The prosthesis 31a also extends medially and is further configured to provide the required coverage of the direct hernia space (floor of the inguinal canal) for a complete inguinal hernia repair.

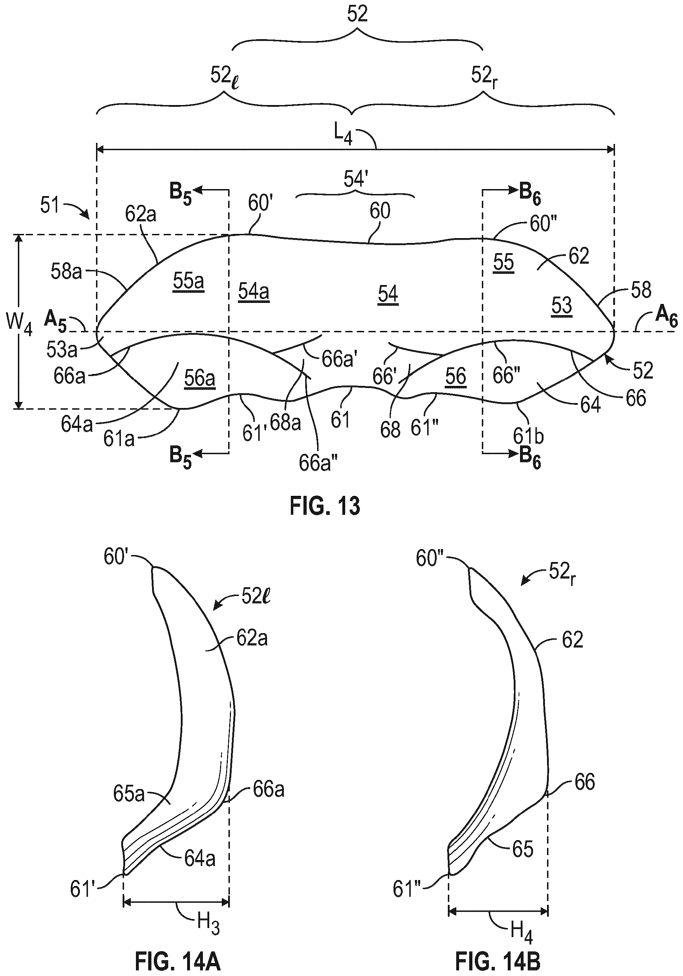

Turning now to Figs. 13-15, an embodiment of an implantable prosthesis 51 including a single biocompatible piece 52, e.g., prosthetic mesh, designed for a bilateral hernia is shown. The prosthesis 51 (and/or piece 52) is designed to anatomically cover both the right and left hernia spaces (bilateral myopectineal orifices). This single piece design allows for increased stability of the prosthesis 51, with less need for fixation and less migration of the prosthesis 51. In some embodiments, however, the prosthesis 51 may be constructed from multiple pieces attached to one another via any suitable attachment means, such as barbs, adhesives, stitching, melt-bonding, and the like. The bilateral hernia prosthesis 51 has a 3D, anatomical shape conforming to the pelvis and bilateral groins, covering bilateral myopectineal orifices as well as the space of Retzius. The bilateral hernia prosthesis 51 is configured to be inserted through a trocar and to later self-deploy back to its intended 3D shape. The bilateral hernia prosthesis 51 can be used to repair two direct, two indirect, or any combination of direct and indirect hernias.

Fig. 13 depicts an implantable prosthesis 51 including a biocompatible piece of material 52 defined by an outer edge 57 and displaying a three-dimensional shape (Figs. 14A and 14B). The outer edge 57 includes a first convex outer lateral edge 58a, a second convex lateral edge 58 opposite the first convex outer lateral edge 58a, a superior outer edge 60, and an inferior outer edge 61 opposite the superior outer edge 60. The inferior outer edge 61 including at least one, if not two or more, concave portion 61', 61".

The biocompatible piece 52 includes a first section 52ₗ and a second section 52ᵣ extending across the face of the piece 52. The first and second sections 52ₗ, 52ᵣ being adjacent to each other across the face of the prosthesis 51.

Fig. 13 depicts a first medial-lateral axis As and a first superior-inferior axis B₅ across the first section 52ₗ of the biocompatible piece 52 (and/or prosthesis 51) and a second medial-lateral axis A₆ and a second superior-inferior axis B₆ across the second section 52ᵣ of the biocompatible piece 52 (and/or prosthesis 51). The first and second medial-lateral axis A₅, A₆ being generally coplanar and the first and second superior-inferior axis B₅, B₆ being generally parallel to each other.

The first section 52ₗ includes a first lateral side 53a, a first medial side 54a, a first superior side 55a and a first inferior side 56a. The second section 52ᵣ includes a second lateral side 53, a second medial side 54, a second superior side 55 and a second inferior side 56. The first medial side 54a of the first section 52ₗ abuts and is attached to the second medial side 54 of the second section 52ᵣ to form a central band 54' of the single and/or one-piece prosthesis 51.

The first section 52ₗ further includes a first upper portion 62a, a first lower portion 64a, a first interior edge 66a and a first crease portion 68a. The first upper portion 62a defines a curved body having a height H₃ transverse to the length and width (Fig. 14A). The first upper portion 62a begins at a first part 60' of the superior outer edge 60 and extends with a raised curved trajectory towards the first interior edge 66a. In some embodiments, the first upper portion 62a may be a spherical cap.

The first lower portion 64a extends between the first interior edge 66a and a first part 61a of the inferior outer edge 61a. The first lower portion 64a forming a first wavy-shaped wall 65a. The first crease portion 68a is defined by the first and second bifurcated end portions 66a', 66a" of the first interior edge 66a on the first medial side 54a.

The second section 52ᵣ further includes a second upper portion 62, a second lower portion 64, a second interior edge 66 and a second crease portion 68. The second upper portion 62 defines a curved body having a height H₄ transverse to the length and width (Fig. 14B). The second upper portion 62 begins at a second part 60" of the superior outer edge 60 and extends with a raised curved trajectory towards the second interior edge 66. In some embodiments, the second upper portion 62 may be a spherical cap.

In some embodiments, the height H₃ of the first section and the height H₄ of the second section may be equal. In some embodiments, the height H₃ of the first section may be greater than the height H₄ of the second section. In some embodiments, the height H₃ of the first section may be less than the height H₄ of the second section.

The second lower portion 64 extends between the second interior edge 66 and a second part 61b of the inferior outer edge 61. The second lower portion 64 forms a second wavy-shaped wall 65. The second crease portion 68 is defined by a third and fourth bifurcated end portions 66', 66" of the second interior edge 66 on the second medial side 54.

As further depicted in Fig. 13, in some embodiments, the biocompatible piece 52 is configured to repair a hernia, such as a direct inguinal hernia, and includes a longest length L₄ and a widest width W₄. The longest length L₄ extends between the first convex lateral outer edge 58a on the first section 52ₗ of the piece 52 and the second opposite convex lateral outer edge 58 on the second section 52ᵣ of the piece 52. The widest width W₄ is located on the first and/or second medial side 54a, 54 of the first and/or second sections 52ₗ, 52ᵣ.

The longest length L₄ of the piece 52 (and/or prosthesis 51) can be between about 28 cm and about 33 cm, and the widest width W₄ of the piece 52 (and/or prosthesis 51) can be between about 10 cm and about 12 cm. For example, a medium size bilateral hernia prosthesis 51 can be about 28 cm x 10 cm (L_{4 x} W₄). A large size bilateral hernia prosthesis 51 can be about 31 cm x 11 cm (L_{4 x} W₄). Finally, an extra-large size bilateral hernia prosthesis 51 can be 33 cm by 12 cm ((L_{4 x} W₄).

In some embodiments, the first interior edge 66a may extend from or near a portion of the first convex lateral edge 58a positioned inferior to the longest length L₄ and/or the second interior edge 66 may extend from or near a portion of the second convex lateral edge 58 positioned inferior to the longest length L₄.

In some embodiments, the first interior edge 66a may extend from or near a portion of the first convex lateral edge 58a positioned along the longest length L₄ and/or the second interior edge 66 may extend from or near a portion of the second convex lateral edge 58 along the longest length L₄.

As further illustrated in Figs. 13 and 14, the first and second lower portions 64a, 64 of the 3D prosthesis 51 on each side can have a raised, wavy section 65a, 65 designed to accommodate the iliac vessels as they course deep (posterior) to the prosthesis 51. The first and second crease portions 58a, 58 allows the lower medial aspect of the prosthesis 51 to sit in the space of Retzius, between the bladder and Cooper's ligament (shown to better advantage in Fig. 15). In addition, the curved body 63', shown in Fig. 14, contributes to the 3D design of the prosthesis 51 and allows for an anatomical fit in the groin.

Fig. 15 illustrates the bilateral hernia prosthesis 51, e.g., surgical mesh, within the anatomical context in which it is designed to be implanted. The prosthesis 51 covers both the left and right myopectineal orifices of the groin, which includes the entire right and lefts inguinal space. The "bilateral" nature of the prosthesis 51 offers preferential coverage both medially over the floor of the inguinal canals and laterally over the internal inguinal rings. The bilateral hernia prosthesis 51 also provides coverage over the right and left femoral canals and can be used for the repair of bilateral femoral hernias.

Turning now to Fig. 16, an embodiment of a bilateral implantable prosthesis 71 including a single biocompatible piece 72, e.g., prosthetic mesh, designed for bilateral indirect hernias is shown. The prosthesis 71 (and/or piece 72) is designed to anatomically cover both the right and left hernia spaces (bilateral myopectineal orifices). This single piece design allows for increased stability of the prosthesis 71, with less need for fixation and less migration of the prosthesis 71. In some embodiments, however, the prosthesis 71 may be constructed from multiple pieces attached to one another via any suitable attachment means, such as barbs, adhesives, stitching, melt-bonding, and the like. The bilateral hernia prosthesis 71 has a 3D, anatomical shape conforming to the pelvis and bilateral groins, covering bilateral myopectineal orifices as well as the space of Retzius. The bilateral indirect hernia prosthesis 71 is configured to be inserted through a trocar and to later self-deploy back to its intended 3D shape. The bilateral indirect hernia prosthesis 71 can be used to repair two indirect hernias.

Fig. 16 depicts an implantable prosthesis 71 including a biocompatible piece of material 72 defined by an outer edge 77 and displaying a three-dimensional shape. The outer edge 77 includes a first convex outer lateral edge 78a, a second convex lateral edge 78 opposite the first convex outer lateral edge 78a, a superior outer edge 80, and an inferior outer edge 81 opposite the superior outer edge 80. The superior outer edge 80 includes at least one, if not two or more, concave portions 80', 80". The inferior outer edge 81 also includes at least one, if not two or more, concave portions 81', 81". In some embodiments, the concave portions of the superior outer edge are aligned with the concave portions of the inferior outer edge along a superior-inferior axis to be symmetrical.

The biocompatible piece 72 includes a first section 72ₗ and a second section 72ᵣ extending across the face of the piece 72. The first and second sections 72ₗ, 72ᵣ being adjacent to each other across the face of the prosthesis 71.

Fig. 16 depicts a first medial-lateral axis A₇ and a first superior-inferior axis B₇ across the first section 72ₗ of the biocompatible piece 72 (and/or prosthesis 71) and a second medial-lateral axis As and a second superior-inferior axis B₈ across the second section 72ᵣ of the biocompatible piece 72 (and/or prosthesis 71). The first and second medial-lateral axis A₇, As being generally coplanar and the first and second superior-inferior axis B₇, B₈ being generally parallel to each other.

The first section 72ₗ includes a first lateral side 73a, a first medial side 74a, a first superior side 75a and a first inferior side 76a. The second section 72ᵣ includes a second lateral side 73, a second medial side 74, a second superior side 75 and a second inferior side 76. The first medial side 74a of the first section 72ₗ abuts and is attached to the second medial side 74 of the second section 72ᵣ to form a central band 74' of the single and/or one-piece prosthesis 71.

The first section 72ₗ further includes a first upper portion 82a, a first lower portion 84a, a first interior edge 86a and a first crease portion 88a. The first upper portion 82a defines a curved body having a height H₃ transverse to the length and width. The first upper portion 82a begins at a first part 80a of the superior outer edge 80 and extends with a raised curved trajectory towards the first interior edge 86a. In some embodiments, the first upper portion 82a may be a spherical cap.

The first lower portion 84a extends between the first interior edge 86a and a first part 81a of the inferior outer edge 81. The first lower portion 84a forming a first wavy-shaped wall. The first crease portion 88a is defined by the first and second bifurcated end portions 86a', 86a" of the first interior edge 86a on the first medial side 74a.

The second section 72ᵣ further includes a second upper portion 82, a second lower portion 84, a second interior edge 86 and a second crease portion 88. The second upper portion 82 defines a curved body having a height H₄ transverse to the length and width. The second upper portion 82 begins at a second part 80b of the superior outer edge 80 and extends with a raised curved trajectory towards the second interior edge 86. In some embodiments, the second upper portion 82 may be a spherical cap.

The second lower portion 84 extends between the second interior edge 86 and a second part 81b of the inferior outer edge 81. The second lower portion 84 forms a second wavy-shaped wall. The second crease portion 88 is defined by a third and fourth bifurcated end portions 86', 86" of the second interior edge 86 on the second medial side 74.

As further depicted in Fig. 16, in some embodiments, the biocompatible piece 72 is configured to repair a hernia, such as an indirect inguinal hernia, and includes a longest length L₅ and a widest width W₅. The longest length L₅ extends between the first convex lateral outer edge 78a on the first section 72ₗ of the piece 72 and the second opposite convex lateral outer edge 78 on the second section 72ᵣ of the piece 72. The widest width W₅ is located on the first and/or second medial side 74a, 74 of the first and/or second sections 72ₗ, 72ᵣ.

The longest length L₅ of the piece 72 (and/or prosthesis 71) can be between about 28 cm and about 33 cm, and the widest width W₅ of the piece 72 (and/or prosthesis 71) can be between about 10 cm and about 12 cm. For example, a medium size bilateral hernia prosthesis 71 can be about 28 cm x 10 cm (L_{5 x} W₅). A large size bilateral hernia prosthesis 71 can be about 31 cm x 11 cm (L_{5 x} W₅). Finally, an extra-large size bilateral hernia prosthesis 71 can be 33 cm by 12 cm (L_{5 x} W₅).

In some embodiments, the first interior edge 86a may extend from or near a portion of the first convex lateral edge 78a positioned inferior to the longest length L₅ and/or the second interior edge 86 may extend from or near a portion of the second convex lateral edge 78 positioned inferior to the longest length L₅.

In some embodiments, the first interior edge 86a may extend from or near a portion of the first convex lateral edge 78a positioned along the longest length L₅ and/or the second interior edge 86 may extend from or near a portion of the second convex lateral edge 78 along the longest length L₅.

In some embodiments, as shown in Fig. 16, a bilateral implantable prosthesis 71 may be configured to treat bilateral indirect hernias. In such embodiments, the first and second sections 72ₗ, 72ᵣ may each be configured similar h as the prostheses 31, 31a, of Figs. 6 and 9. The bilateral indirect prosthesis 71 is defined by an outer edge 71 including a superior outer edge 80 including two or more concave edges, a wavy inferior outer edge 81 and two opposite convex lateral edges 78a, 78.

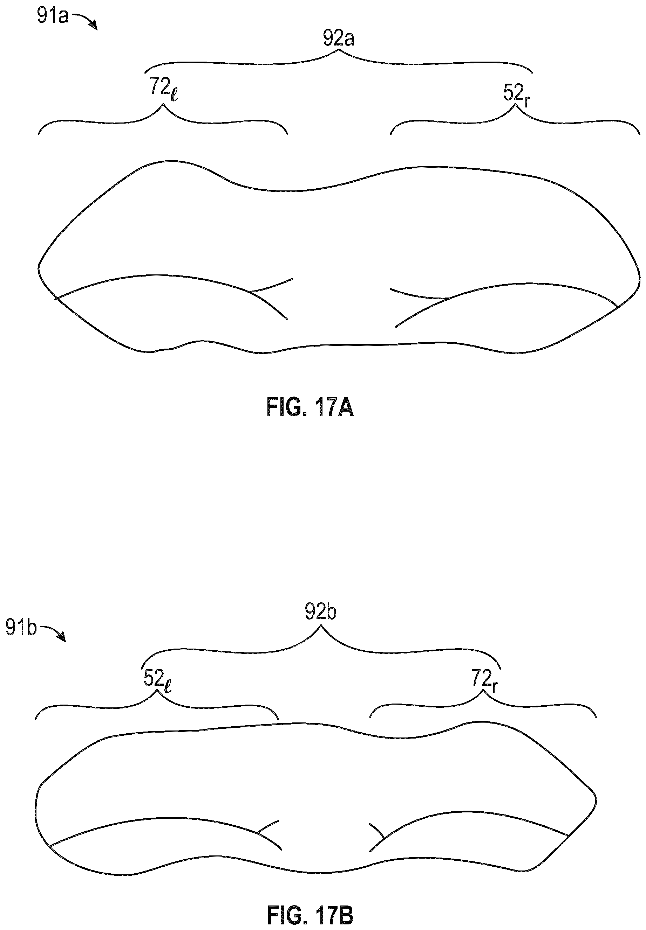

In some embodiments, as shown in Fig. 17A, a bilateral implantable prosthesis 91a configured to treat an indirect hernia and a direct hernia on opposite sides of a patient is shown. The prosthesis 91a may include a first section 72ₗ configured to treat the indirect hernia (as shown and described herein with reference to Fig. 16) and a second section 52ᵣ configured to treat a direct hernia (as shown and described herein with reference to Fig. 13). Each section being largely similar to the first and second sections 72ₗ, 52ᵣ of Figs. 16 and 15, respectively.

In some embodiments, as shown in Fig. 17B, a bilateral implantable prosthesis 91b configured to treat a direct hernia and an indirect hernia on opposite sides of a patient is shown. The prosthesis 91b may include a first section 52ₗ configured to treat a direct hernia (as shown and described herein with reference to Fig. 13) and a second section 72ᵣ configured to treat an indirect hernia (as shown and described herein with reference to Fig. 16). Each section being largely similar to the first and second sections 52ₗ, 72ᵣ of Figs. 15 and 16, respectively.

The prostheses described herein may further include a fastening means. The fastening means can be chosen from among elements that are integrally formed on the prosthesis, such as barbs, loops, and/or hooks. The fastening means designed to attach the prosthesis directly to tissue and/or to another prosthesis.

In some embodiments, the prostheses described herein may include one or more barbs. The barbs can be formed from yarns or portions of yarns that are woven and/or knitted directly with the yarns or portions of yarns used to form the prosthesis. One examples of such barbs is described in WO01/81667. Other examples for barbs, loops and hooks are also available. The one or more barbs may be positioned on any portion of the prostheses described herein.

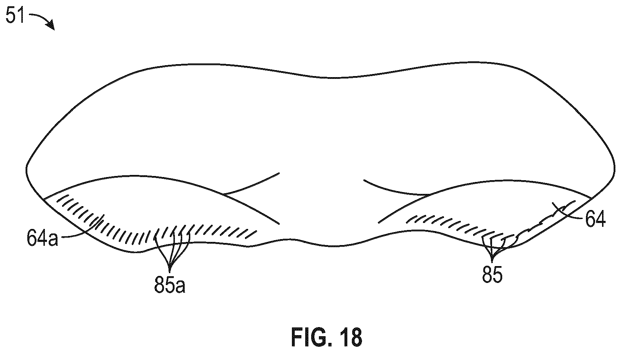

As shown in Fig. 18, for example, in some embodiments one or more barbs 85a, 85 may be positioned on at least one, if not both, of the lower portions 64a, 64 of bilateral prosthesis 51. In other examples, the one or more barbs may be positioned on any of the lower portions 14, 14a, 44, 44a, 64, 64a, of any of the prostheses 1, 1a, 31, 31a, 51, 71, 91a, 91b (as shown in Figs. 1, 4, 7, 10, 13, 16, 17A, 17B) described herein.

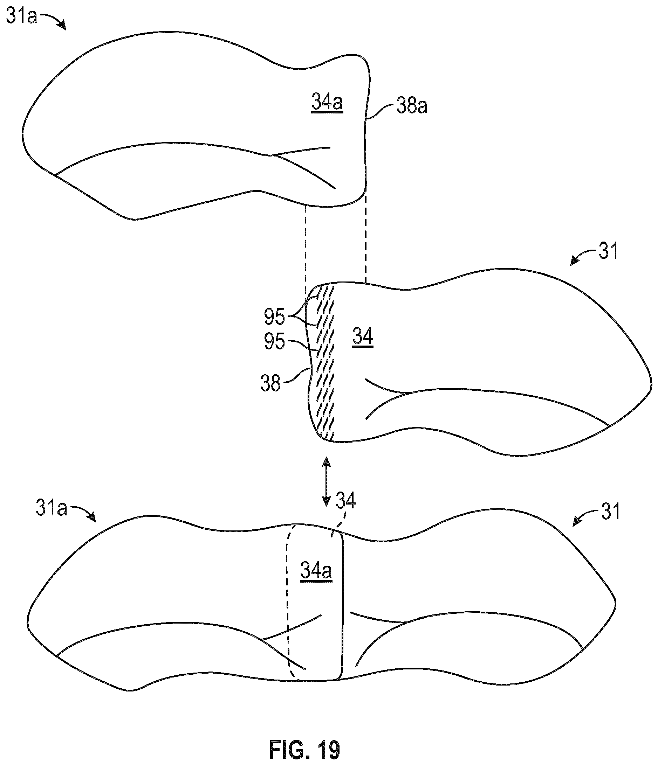

As shown in Fig. 19, in some embodiments the one or more barbs 95 may be positioned on a first unilateral prosthesis. For example, the right-sided indirect prosthesis 31 of Fig. 7 may further include one or more barbs 95 on a medial side 34 and/or along a medial outer edge 39 thereof. In such embodiments, a second unilateral prosthesis, for example left sided indirect prosthesis 31a of Fig. 10, may be combined with the first unilateral prosthesis, e.g., right-sided indirect prosthesis 31, by overlapping the medial sides 34, 34a of each prosthesis such that the barb(s) 95 fasten the two medial sides 34, 43a of the prostheses 31, 31a to form a two-piece bilateral prosthesis. In other examples, the one or more barbs may be positioned on the medial side and/or along the medial outer edge of any of the unilateral prostheses 1, 1a, 31, 31a described herein, and combined with any other unilateral prostheses 1, 1a, 31, 31a described herein to form a two-piece bilateral prosthesis. The two-piece bilateral prosthesis maintains the ability to be separated back into two separate unilateral prostheses, if needed.

Although several embodiments have been described above with a certain degree of particularity, those skilled in the art could make numerous alterations to the disclosed embodiments It is intended that all matter contained in the above description or shown in the accompanying drawings shall be interpreted as illustrative only and not limiting. Changes in detail or structure may be made without departing from the present teachings.

Various embodiments are described herein of various apparatuses, systems, and methods. Numerous specific details are set forth to provide a thorough understanding of the overall structure, function, manufacture, and use of the embodiments as described in the specification and illustrated in the accompanying drawings. It will be understood by those skilled in the art, however, that the embodiments may be practiced without such specific details. In other instances, well-known operations, components, and elements have not been described in detail so as not to obscure the embodiments described in the specification. Those of ordinary skill in the art will understand that the embodiments described and illustrated herein are non-limiting examples, and thus it can be appreciated that the specific structural and functional details disclosed herein may be representative and do not necessarily limit the scope of the embodiments, the scope of which is defined solely by the appended claims.

Reference throughout the specification to "various embodiments," "some embodiments," "one embodiment," "an embodiment," or the like, means that a particular feature, structure, or characteristic described in connection with the embodiment is included in at least one embodiment. Thus, appearances of the phrases "in various embodiments," "in some embodiments," "in one embodiment," "in an embodiment," or the like, in places throughout the specification are not necessarily all referring to the same embodiment. Furthermore, the particular features, structures, or characteristics may be combined in any suitable manner in one or more embodiments. Thus, the particular features, structures, or characteristics illustrated or described in connection with one embodiment may be combined, in whole or in part, with the features, structures, or characteristics of one or more other embodiments without limitation.

Unless explained otherwise, all technical and scientific terms used herein have the same meaning as commonly understood to one of ordinary skill in the art to which this disclosure belongs. It will be appreciated that the terms "medial" and "lateral" may be used throughout the specification with reference to portions of an implantable prosthesis. The term "medial" refers to a portion of the implantable prosthetic or the body that lies or extends toward the median axis of the body, and the term "lateral" refers to a portion of the implantable prosthetic or the body that lies or extends toward the side. It will be further appreciated that for conciseness and clarity, anatomical spatial terms such as "superior," "inferior," "anterior," and "posterior" may be used herein with respect to the illustrated embodiments. However, implantable prosthetics may be used in many orientations and positions, and these terms are not intended to be limiting and absolute.

To provide a more concise description, some of the quantitative expressions given herein are not qualified with the term "about." It is understood that whether the term "about" is used explicitly or not, every quantity given herein is meant to refer to the actual given value, and it is also meant to refer to the approximation to such given value that would reasonably be inferred based on the ordinary skill in the art, including approximations due to the experimental and/or measurement conditions for such given value.

## Claims

1. An implantable prosthesis (1) for repairing a direct or indirect inguinal hernial defect comprising:
a piece of biocompatible material (2) having a preformed three-dimensional shape and defined by an outer edge (7) , the piece having a lateral side (3) and an opposite medial side (4) at opposite ends of a medial-lateral axis, and a superior side (5) and an opposite inferior side (6) at opposite ends of a superior-inferior axis, and the outer edge including a convex lateral outer edge (8), a medial outer edge (9), a superior outer edge (10)configured to widen the piece in a medial direction, and an inferior outer edge (11) including one or more concave portions, the piece including:
an upper portion (12) including a spherical cap extending between the superior outer edge and an interior edge defining a crease or fold extending from the lateral side to the medial side, the interior edge including first and second bifurcated portions (46', 46") on a medial end thereof,
a lower portion (14) extending between the interior edge and the inferior outer edge, the lower portion forming a wavy-shaped wall (15), and
a crease portion (18) on the medial side, the crease portion defined between the first and second bifurcated end portions on the medial end thereof, **characterized in that** at least one of the first and second bifurcated portions (46', 46") of the interior edge are non-linear and do not extend to the medial outer edge.

2. The implantable prosthesis according to claim 1, wherein superior outer edge (10) is generally linear.

3. The implantable prosthesis according to claim 2, wherein the piece further comprises a longest length and a widest width, the longest length extending between the convex lateral outer edge (8) and the medial outer edge (9) and the widest width located on the medial side of the piece extending between the superior outer edge (10) and the inferior outer edge (11).

4. The implantable prosthesis according to claim 3, wherein the interior edge (16) extends from a portion of the convex lateral edge (8) positioned inferior to the longest length.

5. The implantable prosthesis according to claim 4, wherein the interior edge (16) is a non-linear interior edge extending along or crossing over at least a portion of the longest length.

6. The implantable prosthesis according to claim 4, wherein the medial outer edge (9) is a concave medial outer edge.

7. The implantable prosthesis according to any preceding claim 1, further comprising one or more barbs (95) positioned on a face of the piece.

8. The implantable prosthesis according to claim 8, wherein the one or more barbs are positioned on at least a portion of the medial side of the piece along the medial outer edge, positioned on at least a portion of the lower portion of the piece along the inferior outer edge, or both.

9. An implantable prosthesis according to claim 1, further comprising a second section (52),
the second section including:
a second lateral side (53) opposite a second medial side (54) extending along a second medial-lateral axis of the second section and a second superior side (55) opposite a second inferior side extending along a second superior-inferior axis of the second section,
a second upper portion (62) including a spherical cap extending between a second portion of the superior outer edge (10) and a second interior edge (66) extending from the second lateral side (53) to the second medial side (54), the second interior edge including a third and fourth bifurcated portion on a medial end thereof,
a second lower portion (64) extending between the second interior edge (66) a second portion of the inferior outer edge (11), the second lower portion forming a second wavy-shaped wall (65),
a second crease portion (68) on the second medial portion and defined between the third and fourth bifurcated portions of the second crease on the medial end thereof,
wherein the first and second medial sides of the first and second sections form a central band (54') between the first and second lateral sides across a face of the piece of biocompatible material.

## Patentansprüche

1. Implantierbare Prothese (1) zum Reparieren eines direkten oder indirekten inguinalen Herniendefekts, umfassend:
ein Stück aus biokompatiblem Material (2) mit einer vorgeformten dreidimensionalen Formgebung und definiert durch eine Außenkante (7), wobei das Stück eine laterale Seite (3) und eine gegenüberliegende mediale Seite (4) an gegenüberliegenden Enden einer medial-lateralen Achse und eine superiore Seite (5) und eine gegenüberliegende inferiore Seite (6) an gegenüberliegenden Enden einer superior-inferioren Achse aufweist, und wobei die Außenkante eine konvexe laterale Außenkante (8), eine mediale Außenkante (9), eine superiore Außenkante (10), die dazu ausgelegt ist, das Stück in einer medialen Richtung zu verbreitern, und eine inferiore Außenkante (11) einschließt, die einen oder mehrere konkave Abschnitte einschließt, wobei das Stück Folgendes einschließt:
einen oberen Abschnitt (12), der eine sphärische Kappe einschließt, die sich zwischen der superioren Außenkante und einer Innenkante erstreckt, und einen Knick oder Falte definiert, der/die sich von der lateralen Seite zu der medialen Seite erstreckt, wobei die Innenkante einen ersten und einen zweiten gegabelten Abschnitt (46', 46") an einem medialen Ende davon einschließt,
einen unteren Abschnitt (14), der sich zwischen der Innenkante und der inferioren Außenkante erstreckt, wobei der untere Abschnitt eine wellenförmige Wand (15) bildet, und
einen Knickabschnitt (18) auf der medialen Seite, wobei der Knickabschnitt zwischen dem ersten und dem zweiten gegabelten Endabschnitt an dessen medialem Ende definiert ist, **dadurch gekennzeichnet, dass** mindestens einer von dem ersten und dem zweiten gegabelten Abschnitt (46', 46") der Innenkante nichtlinear ist und sich nicht zu der medialen Außenkante erstreckt.

2. Implantierbare Prothese nach Anspruch 1, wobei die superiore Außenkante (10) allgemein linear ist.

3. Implantierbare Prothese nach Anspruch 2, wobei das Stück ferner eine längste Länge und eine breiteste Breite umfasst, wobei sich die längste Länge zwischen der konvexen lateralen Außenkante (8) und der medialen Außenkante (9) erstreckt und sich die breiteste Breite auf der medialen Seite des Stücks befindet und sich zwischen der superioren Außenkante (10) und der inferioren Außenkante (11) erstreckt.

4. Implantierbare Prothese nach Anspruch 3, wobei sich die Innenkante (16) von einem Abschnitt der konvexen lateralen Kante (8) erstreckt, der inferior zu der längsten Länge positioniert ist.

5. Implantierbare Prothese nach Anspruch 4, wobei die Innenkante (16) eine nichtlineare Innenkante ist, die sich entlang mindestens eines Abschnitts der längsten Länge erstreckt oder diesen überkreuzt.

6. Implantierbare Prothese nach Anspruch 4, wobei die mediale Außenkante (9) eine konkave mediale Außenkante ist.

7. Implantierbare Prothese nach einem der vorhergehenden Ansprüche 1, die ferner einen oder mehrere Widerhaken (95) umfasst, der/die auf einer Fläche des Stücks positioniert ist/sind.

8. Implantierbare Prothese nach Anspruch 8, wobei der eine oder die mehreren Widerhaken an mindestens einem Abschnitt der medialen Seite des Stücks entlang der medialen Außenkante positioniert ist/sind, an mindestens einem Abschnitt des unteren Abschnitts des Stücks entlang der inferioren Außenkante positioniert ist/sind, oder beides.

9. Implantierbare Prothese nach Anspruch 1, die ferner ein zweites Segment (52) umfasst,
wobei das zweite Segment Folgendes einschließt:
eine zweite laterale Seite (53) gegenüber einer zweiten medialen Seite (54), die sich entlang einer zweiten medial-lateralen Achse des zweiten Segments erstreckt, und eine zweite superiore Seite (55) gegenüber einer zweiten inferioren Seite, die sich entlang einer zweiten superior-inferioren Achse des zweiten Segments erstreckt,
einen zweiten oberen Abschnitt (62), der eine sphärische Kappe einschließt, die sich zwischen einem zweiten Abschnitt der superioren Außenkante (10) und einer zweiten Innenkante (66) erstreckt, die sich von der zweiten lateralen Seite (53) zu der zweiten medialen Seite (54) erstreckt, wobei die zweite Innenkante einen dritten und vierten gegabelten Abschnitt an einem medialen Ende davon einschließt,
einen zweiten unteren Abschnitt (64), der sich zwischen der zweiten Innenkante (66) und einem zweiten Abschnitt der inferioren Außenkante (11) erstreckt, wobei der zweite untere Abschnitt eine zweite wellenförmige Wand (65) bildet,
einen zweiten Knickabschnitt (68) auf dem zweiten medialen Abschnitt und definiert zwischen dem dritten und dem vierten gegabelten Abschnitt der zweiten Knickstelle an dem medialen Ende davon,
wobei die erste und die zweite mediale Seite des ersten und des zweiten Segments ein zentrales Band (54') zwischen der ersten und der zweiten lateralen Seite über eine Fläche des Stücks aus biokompatiblem Material bilden.

## Revendications

1. Prothèse implantable (1) pour réparer un défaut herniaire inguinal direct ou indirect comprenant :
une pièce de matériau biocompatible (2) ayant une forme tridimensionnelle préformée et définie par un bord extérieur (7), la pièce ayant un côté latéral (3) et un côté médial opposé (4) aux extrémités opposées d'un axe médial-latéral, et un côté supérieur (5) et un côté inférieur opposé (6) aux extrémités opposées d'un axe supérieur-inférieur, et le bord extérieur comportant un bord extérieur latéral convexe (8), un bord extérieur médial (9), un bord extérieur supérieur (10) configuré pour élargir la pièce dans une direction médiale, et un bord extérieur inférieur (11) comprenant une ou plusieurs parties concaves, la pièce comportant :
une partie supérieure (12) comprenant une calotte sphérique s'étendant entre le bord extérieur supérieur et un bord intérieur définissant un pli ou une plicature s'étendant du côté latéral au côté médial, le bord intérieur comprenant des première et deuxième parties bifurquées (46', 46") sur une extrémité médiale de celle-ci,
une partie inférieure (14) s'étendant entre le bord intérieur et le bord extérieur inférieur, la partie inférieure formant une paroi ondulée (15), et
une partie de pli (18) sur le côté médial, la partie de pli étant définie entre les première et seconde parties d'extrémité bifurquées sur l'extrémité médiale de celle-ci, **caractérisée en ce qu'**au moins une des première et seconde parties bifurquées (46', 46") du bord intérieur sont non linéaires et ne s'étendent pas jusqu'au bord extérieur médial.

2. Prothèse implantable selon la revendication 1, le bord extérieur supérieur (10) étant généralement linéaire.

3. Prothèse implantable selon la revendication 2, la pièce comprenant en outre une longueur la plus longue et une largeur la plus large, la longueur la plus longue s'étendant entre le bord extérieur latéral convexe (8) et le bord extérieur médial (9) et la largeur la plus large située sur le côté médial de la pièce s'étendant entre le bord extérieur supérieur (10) et le bord extérieur inférieur (11).

4. Prothèse implantable selon la revendication 3, le bord intérieur (16) s'étendant à partir d'une partie du bord latéral convexe (8) positionnée en dessous de la longueur la plus longue.

5. Prothèse implantable selon la revendication 4, le bord intérieur (16) étant un bord intérieur non linéaire s'étendant le long ou se croisant sur au moins une partie de la longueur la plus longue.

6. Prothèse implantable selon la revendication 4, le bord extérieur médial (9) étant un bord extérieur médial concave.

7. Prothèse implantable selon l'une quelconque des revendications précédentes 1, comprenant en outre un ou plusieurs picots (95) positionnés sur une face de la pièce.

8. Prothèse implantable selon la revendication 8, le ou les picots étant positionnés sur au moins une partie du côté médial de la pièce le long du bord extérieur médial, positionnés sur au moins une partie de la partie inférieure de la pièce le long du bord extérieur inférieur, ou les deux.

9. Prothèse implantable selon la revendication 1, comprenant en outre une seconde section (52),
la seconde section comportant :
un second côté latéral (53) opposé à un second côté médial (54) s'étendant le long d'un second axe médial-latéral de la seconde section et un second côté supérieur (55) opposé à un second côté inférieur s'étendant le long d'un second axe supérieur inférieur de la seconde section,
une deuxième partie supérieure (62) comprenant une calotte sphérique s'étendant entre une deuxième partie du bord extérieur supérieur (10) et un second bord intérieur (66) s'étendant du second côté latéral (53) au second côté médial (54), le second bord intérieur comprenant une troisième et une quatrième parties bifurquées sur une extrémité médiale de celui-ci,
une deuxième partie inférieure (64) s'étendant entre le second bord intérieur (66) une deuxième partie du bord extérieur inférieur (11), la deuxième partie inférieure formant une seconde paroi ondulée (65),
une deuxième partie de pli (68) sur la deuxième partie médiale et définie entre les troisième et quatrième parties bifurquées du second pli sur son extrémité médiale,
les premier et second côtés médiaux des première et seconde sections formant une bande centrale (54') entre les premier et second côtés latéraux sur une face de la pièce de matériau biocompatible.
